(19) Europäisches Patentamt
European Patent Office
Office européen des brevets

(11) EP 1 311 249 B1

(12) **EUROPÄISCHE PATENTSCHRIFT**

(45) Veröffentlichungstag und Bekanntmachung des Hinweises auf die Patenterteilung:
**07.10.2009 Patentblatt 2009/41**

(21) Anmeldenummer: **01978306.7**

(22) Anmeldetag: **24.08.2001**

(51) Int Cl.:
***A61K 9/70*** *(2006.01)* ***A61K 31/48*** *(2006.01)*
***A61P 5/08*** *(2006.01)* ***A61P 5/24*** *(2006.01)*

(86) Internationale Anmeldenummer:
**PCT/EP2001/009824**

(87) Internationale Veröffentlichungsnummer:
**WO 2002/015890 (28.02.2002 Gazette 2002/09)**

(54) **SET AUS TRANSDERMALEN THERAPEUTISCHEN SYSTEMEN**

SET OF TRANSDERMAL THERAPEUTIC SYSTEMS

ASSOCIATION DE SYSTEMES THERAPEUTIQUES TRANSDERMIQUES

(84) Benannte Vertragsstaaten:
**AT BE CH CY DE DK ES FI FR GB GR IE IT LI LU MC NL PT SE TR**

(30) Priorität: **24.08.2000 DE 10043321**

(43) Veröffentlichungstag der Anmeldung:
**21.05.2003 Patentblatt 2003/21**

(73) Patentinhaber: **Axxonis Pharma AG**
**10963 Berlin (DE)**

(72) Erfinder:
- **WINDT-HANKE, Fred**
  **12157 Berlin (DE)**
- **GÜNTHER, Clemens**
  **13357 Berlin (DE)**
- **HOROWSKI, Reinhard**
  **14129 Berlin (DE)**
- **TACK, Johannes**
  **13595 Berlin (DE)**

(74) Vertreter: **Wablat, Wolfgang et al**
**Patentanwalt**
**Dr. Dr. W. Wablat**
**Potsdamer Chaussee 48**
**14129 Berlin (DE)**

(56) Entgegenhaltungen:

| | |
|---|---|
| **EP-A- 0 137 278** | **EP-A- 1 027 889** |
| **DE-A- 4 116 912** | **US-A- 5 229 129** |
| **US-A- 5 399 355** | **US-A- 5 696 128** |

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Set aus transdermalen therapeutischen Systemen (TTS-Set), wobei das Set eine Mehrzahl von TTS-Elementen enthält und wobei die Elemente zur Abgabe von unterschiedlichen Dosen eingerichtet sind, zur Erreichung und Erhaltung des circadianen Rhythmus unter kontinuierlicher dopaminerger Therapie.

Der Begriff des TTS umfasst insbesondere perkutan wirkende, aber auch transmucosal wirkende Systeme. Ein TTS ist typischerweise von flächiger Struktur und wird beispielsweise auf der Haut flächig zum Anliegen gebracht. Die Befestigung auf der Haut kann durch ein ggf. zusätzliches hautseitiges (und für den Wirkstoff permeables) Adhäsiv erfolgen. Ebenso kann die Matrix und/oder die Diffusionsbarriere selbst mit adhäsiven Eigenschaften ausgestattet sein. Schließlich kann ein nicht adhäsives TTS mittels weiterer Hilfsmittel, beispielsweise Klebebänder oder Bandagen, auf der Haut zum Anliegen gebracht werden. Als Matrix ist ein Stoff bezeichnet, in welchem der Wirkstoff immobilisiert ist. Demgegenüber ist der Wirkstoff in einem Wirkstoffreservoir nicht notwendigerweise immobilisiert, weswegen das Wirkstoffreservoir ummantelt sein muss. Der hautseitige Teil des Mantels wird dabei von der Diffusionsbarriere gebildet. Es versteht sich, dass der weitere Teil des Mantels möglichst inpermeabel, auch bezüglich Diffusionspfade, für den Wirkstoff sein sollte. Der Begriff immobilisiert meint in diesen Zusammenhängen, dass kein unkontrollierter Wirkstoff-Fluss möglich ist.

Insbesondere Diffusion eines Wirkstoffes in einer Matrix und/oder durch eine Diffusionsbarriere ist jedoch nicht nur möglich, sondern gezielt eingerichtet. Die Diffusionskoeffizienten bestimmen dabei letztendlich den Flux des Wirkstoffes aus dem TTS in die Haut eines Patienten. Die an die Haut eines Patienten abgegebene Dosis ist daher eine in erster Näherung lineare Funktion der wirksamen Fläche des TTS. Die wirksame Fläche ist die Kontaktfläche von für Wirkstoffe diffusionsoffenen Bereichen des TTS. TTS sind sowohl für humane als auch für veterinärmedizinische Zwecke einsetzbar.

Ein TTS des eingangs genannten Aufbaus ist grundsätzlich bekannt aus der Literaturstelle WO 92/20339. Hierin ist insbesondere der Effekt von Propylenglycol-Laurinsäure auf den Flux beschrieben, wodurch eine beachtliche Fluxerhöhung erreicht wird.

Die angegebenen Werte beziehen sich dabei auf auf Hautproben aufgetragene Lösungen und nicht auf die eigentlichen TTS. Zu diesen sind keinerlei Angabe hinsichtlich des Flux gemacht.

Ein Lisurid enthaltendes TTS ist weiterhin bekannt aus der Literaturstelle WO 91/00746. Die darin angegebenen Flux-Werte für menschliche Hautproben sind nicht ohne weiteres auf erzielbare in vivo Werte übertragbar.

TTS des beschriebenen Aufbaus werden für verschiedene Indikation, u. a. Parkinson, verwendet. Im Fall einer Behandlung der Parkinsonschen Krankheit sind möglichst hohe Dosen wünschenswert. Ein transdermales therapeutisches System verbessert hier zusätzlich die Compliance, die für die Kombinationstherapien dieser Krankheit und ihren meist alten und multimorbiden Patienten von sehr erheblicher Bedeutung ist. Eine bessere Steuerbarkeit sowie die Möglichkeit, circadiane Profile zu erreichen (z. B. mit geringer, möglichst konstanter Stimulation während der Nacht bzw. einer Pause) sind hier besonders wichtig und bisher nicht erreicht (z. B. zur Vermeidung von Psychosen sowie zur Verbesserung der Schlafqualität).

Im Falle der Ergolin-Derivate der Formel I trägt auch deren dopamin-partialagonistische bzw. partialantagonistische Wirkung dazu bei, das Entstehen von Psychosen zu verhindern bzw. vorhandene Psychosen und ähnliche Probleme zu bessern.

Bei der Parkinson Therapie, bei der dopaminerge Pharmaka und ihre Kombinationen über den Tag verteilt eingenommen werden, ist die Plasmakonzentration nicht konstant, sondern grossen Schwankungen unterworfen, und zwar nicht nur aus kinetischen Gründen (sehr variabler First-Pass-Effekt in Abhängigkeit vom Metabolisierungstyp), sondern auch abhängig von den individuellen Randbedingungen der Einnahme (Art und Zeitpunkt der Nahrungsaufnahme, Wirkung anderer Pharmaka auf die Resorption und den Metabolismus etc.). Daher besteht auch die Gefahr einer zeitweisen Überdosierung mit der Folge von beispielsweise einer REM Unterdrückung und daraus resultierenden Schlafstörungen oder Psychosen.

Außerdem sind bei den bisherigen dopaminergen Therapien langanhaltende und schwerwiegende Nebenwirkungen häufig. Hier kann mit einem transdermalen therapeutischen System gemäss der nachfolgend beschriebenen Erfindung eine auch individuell dosierbare, gut steuerbare und kontrollierte Wirkdauer (ggf. durch Entfernen des Pflasters) erreicht werden, ohne den circadianen Rhythmus zu beeinflussen, der häufig in Folge der Therapie bei Morbus Parkinson und bei anderen dopaminergen Erkrankungen gestört ist.

Der Erfindung liegt das technische Problem zugrunde, ein Mittel zur Erreichung und Erhaltung des circadianen Rhythmus zur Verfügung zu stellen, das individuell dosierbar, gut steuerbar und in der Wirkdauer gut kontrollierbar ist und somit die circadianen Störungen, die unter dopaminerger Therapie bei der Behandlung von dopaminergen Erkrankungen, insbesondere bei der Behandlung von Parkinson Patienten, auftreten, verhindert. Die $\alpha$-adrenolytische Wirkung von Lisurid und den Ergolin-Derivaten der Formel I hat bei dieser Form der Anwendung den weiteren Vorzug, dass auch nächtlicher Harndrang und andere Blasen-Funktionsstörungen, wie dies bei Parkinsonpatienten nicht selten ist (u. a. auch durch Prostatahyperplasie), deutlich gebessert werden, was gleichfalls zum Therapieerfolg beiträgt.

Zur Lösung dieses technischen Problems lehrt die Erfindung die Verwendung eines Sets aus transdermalen therapeutischen Systemen (TTS-Set), wobei das Set eine Mehrzahl von TTS-Elementen enthält und wobei die Elemente zur Abgabe von unterschiedlichen Dosen eingerichtet sind, wobei das transdermale therapeutische System (TTS) eine Arzneimittelschicht aufweist, welche zumindest eine einen Wirkstoff enthaltende Matrix und/oder ein Wirkstoffreservoir und hautseitig des Wirkstoffreservoirs eine wirkstoffpermeable Diffusionsbarriere sowie als Wirkstoff ein Ergolin-Derivat gemäss Formel I oder dessen physiologisch verträgliches Salz mit einer Säure enthält,

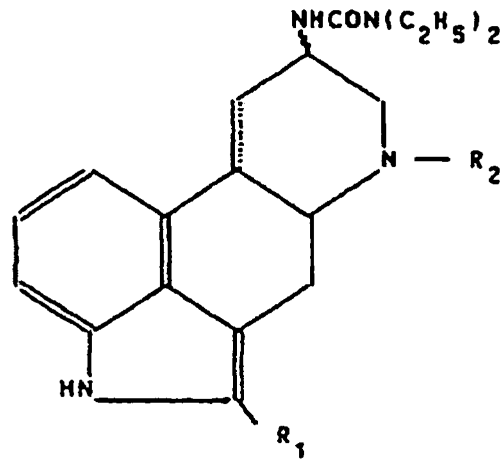

Formel I

worin ------ eine Einfachbindung oder eine Doppelbindung ist, worin R1 ein H-Atom oder ein Halogenatom, insbesondere ein Bromatom ist, und worin R2 C1-4-Alkyl ist, zur Erreichung und Erhaltung des circadianen Rhythmus unter kontinuierlicher dopaminerger Therapie,
wobei die Matrix und/oder die Diffusionsbarriere ausgewählt ist mit der Massgabe, dass der transdermale Flux F durch Humanhaut im Bereich von 0,1 bis 5,0 $\mu g/cm^2/h$ liegt.

In Frage kommende Salze der Wirkstoffe sind beispielsweise Sulfate, Phosphate, Maleate, Citrate und Succinate sowie insbesondere Hydrogenmaleat.

Die Erfindung beruht auf der überraschenden Erkenntnis, dass mit einem Ergolinderivat der Formel I oder dessen Salz, das hocheffektiv ist und eine kurze Halbwertszeit (0,5 bis 4 Stunden, insbesondere 1 bis 2 Stunden) aufweist, circadiane Störungen unter dopaminergen Therapien vermieden werden. Mit der Erfindung wird als besonderer Vorteil erreicht, dass ein kontinuierlicher Wirkstofflux eingerichtet wird und somit die Plasmakonzentrationen sich definiert einstellen und hinsichtlich des Verlaufes kontrollieren lässt. Dies verhindert weitgehend die bei oralen Einmalgaben und bei TTS enthaltend Wirkstoffe mit langer Halbwertszeit zu beobachtenden dopaminergen Nebenwirkungen, wie Müdigkeit, Schwindel etc. Denn es hat sich herausgestellt, dass diese Nebenwirkungen sich vermeiden lassen, wenn die Plasma Konzentrationen des Wirkstoffes nicht grossen und schnellen Schwankungen unterworfen werden, wie bei oraler Gabe sich automatisch einstellend, sondern langsam und kontinuierlich eingestellt werden. Hinzu kommt, dass die Problematik oraler Gaben, wie stark veränderliche Absorptionsgeschwindigkeiten und wenig definierter Zeitpunkt der Maximalkonzentration im Plasma, beispielsweise abhängig von Art und Zeitpunkt der Nahrungsaufnahme, mit der Erfindung praktisch eliminiert werden. Insbesondere eine Überdosierung (und folglich REM-Unterdrückung sowie andere Schlafmusterstörungen) wird vermieden. Weiterhin kann vergleichsweise schnell abgesetzt werden, nämlich einfach durch Entfernung des TTS. Auf Grund der erfindungsgemäss geeigneten Wirkstoffe mit kurzer Halbwertszeit wird der Abfall der Plasmakonzentration an Wirkstoff bei Abnahme des TTS zusätzlich beschleunigt. Im Gegensatz zum Absetzen eines oral verabreichten Wirkstoffs oder eines Wirkstoffes mit langer Halbwertszeit erfolgt der Abbau im Plasma zügig und kontrolliert, wodurch auch "hang over" vermieden werden kann. Schliesslich ist eine exakte individuelle Dosierung durch Auswahl des Flux F und/oder der wirksamen Fläche unschwer möglich. Vorzugsweise werden F und wirksame Fläche so ausgewählt, dass sich am zweiten Tag der Applikation eine effektive Dosis im Bereich von 10 $\mu g$ bis 2 mg Wirkstoff, vorzugsweise 50 $\mu g$ bis 1 mg, (beispielsweise Lisurid) über den Tag bzw. 24 Stunden im Organismus einstellt.

Bevorzugt ist es, wenn die Matrix und/oder die Diffusionsbarriere ausgewählt ist mit der Massgabe, dass der transdermale Flux F durch Humanhaut, gemessen gemäss Beispiel 1, im Bereich von 0,1 bis 5,0 $\mu g/cm^2/h$, insbesondere von 0,5 bis 2,5 $\mu g/cm^2/h$ liegt. Ein Pflaster dieser Spezifikation ist besonders geeignet, mit dem Wirkstoff Lisurid kontinuierliche Plasmakonzentrationen im Bereich von 0,05 bis 5,0 ng/ml, vorzugsweise von 0,1 bis 0,5 ng/ml zu erzielen.

Insbesondere bevorzugt ist die Verwendung eines TTS aufweisend eine Matrix und als Wirkstoff ein Ergolinderivat der Formel I oder dessen Salz.

Als Ergolinderivate kommen beispielsweise in Frage: Bromlisurid (3- (2-Brom-9, 10-didehydro-6-methyl-8α-ergolinyl)-1, 1-diethylharnstoff), Tergurid (3- (6- methyl-8α-ergolinyl)-1, 1-diethylharnstoff) und Protergurid (3- (6-propyl-8a-ergolinyl)-1, 1-diethylharnstoff). Bevorzugt ist es allerdings, wenn das Ergolin-Derivat Lisurid (3-(9, 10-didehydro-6-methyl- 8α-ergolinyl)-1, 1-diethylharnstoff) oder dessen physiologisch verträgliches Salz mit einer Säure ist.

Die Herstellung von Lisurid und den weiteren erfindungsgemäss geeigneten Ergolinen wird beispielsweise in US 3,953,454, EP 056 358 und US 4,379,790 beschrieben. In Frage kommende Salze des Ergolin-Derivats sind beispielsweise Sulfate, Phosphate, Maleate, Citrate und Succinate sowie insbesondere Hydrogenmaleat.

Das TTS kann im einzelnen wie folgt ausgebildet sein. Auf der hautabgewandten Seite der Matrix und/oder des Wirkstoffreservoirs kann eine Deckschicht angeordnet sein. Diese kann beispielsweise mit Folien aus Polyethylen oder Polyester gebildet sein. Die Dicke beträgt typischerweise 10 bis 100 μm. Es ist möglich zur Erzielung eines ausreichenden Lichtschutzes, die Deckschicht zu pigmentieren und/oder zu metallisieren.

Als Metallisierung ist das Aufbringen einer sehr dünnen Schicht (typischerweise weniger als 1 μm, meist im 10-100 nm Bereich) eines Metalls, beispielsweise Aluminium, auf die Deckschicht bezeichnet. Pigmente können alle im Rahmen der Überzugsmittel gebräuchlichen Pigmente, auch Effektpigmente, sein, sofern sie physiologisch unbedenklich sind. Auf der Applikationsseite kann ein abziehbarer Liner vorgesehen sein, beispielsweise eine silikonisierte oder fluorpolymerbeschichtete polymere Schutzfolie.

Die Matrix und/oder Diffusionsbarriere kann einen Stoff, ausgewählt aus der Gruppe bestehend aus Polyacrylat, Polyurethan, Celluloseether, Silikon, Polyvinylverbindungen, Silikat und Mischungen dieser Stoffe sowie Copolymere dieser Polymerverbindungen, vorzugsweise hydrophiles Polyacrylat mit basischen Substituenten, als Hauptmatrixkomponente aufweisen.

Eine Hauptmatrixkomponente bildet zumindest 50 Gew.-%, beispielsweise zumindest 80-90 Gew.-% der Matrix (der Begriff der Matrix bezieht sich dabei auf die fertige Schicht, i. e. Hauptmatrixkomponente (n) mit Hilfsstoff (en) und Wirkstoff (en)). Eine Einstellung des gewünschten Flux erfolgt einerseits durch Auswahl des Stoffes in Abhängigkeit des Diffusionskoeffizienten des Wirkstoffes darin und andererseits und ggf. in Abstimmung hiermit durch Wahl der Schichtdicke der Matrix in Richtung orthogonal zur Hautoberfläche. Der Dickenbereich einer Matrix liegt typischerweise im Bereich von 10 μm bis 500 μm.

Ein bevorzugter Polyacrylatkleber als Hauptmatrixkomponente ist käuflich unter der Bezeichnung GELVA® multipolymer solution 7881, erhältlich von der Firma Monsanto Deutschland GmbH, Düsseldorf. Dabei wird ausdrücklich Bezug genommen auf das unter dieser Bezeichnung vertriebene Produkt gemäss Datenblatt in der Fassung vom 23.04.1996. Besonders gut verwendbar ist Eudragit® E100, erhältlich von der Firma Röhm, Deutschland, das ein Copolymerisat aus Dimethylaminomethylmethacrylat mit neutralen Methacrylsäureestern ist.

Mit den vorstehenden Polyacrylatklebern wird eine besonders vorteilhafte nichttriviale Eigenschaftskombination erhalten, nämlich optimaler Flux, gute Haftfähigkeit, gute Hautverträglichkeit und gute Haltbarkeit.

Die Diffusionsbarriere kann alternativ ein Polymer ausgewählt aus der Gruppe bestehend aus Celluloseester, Celluloseether, Silikon, Polyolefin und Mischungen sowie Copolymere dieser Stoffe als Hauptbarrierenkomponente aufweisen. Zum Begriff der Hauptbarrierenkomponente gilt das Vorstehende zur Hauptmatrixkomponente analog. Die Diffusionsbarriere kann als Folie mit einer Dicke von 10 μm bis 300 μm ausgebildet sein, wobei die Dicke der Schicht (in Verbindung mit dem Diffusionskoeffizienten des Wirkstoffes in dem Polymer) nach Massgabe des gewünschten Flux eingestellt wird.

In der Matrix und/oder dem Wirkstoffreservoir und/oder der Diffusionbarriere können für TTS übliche Hilfsstoffe enthalten sein. Bevorzugterweise wird als Hilfsstoff ein penetrationsverstärkendes Mittel eingesetzt, welches vorzugsweise ausgewählt ist aus der Gruppe bestehend aus C1-C8 aliphatische, cycloaliphatische und aromatische Alkohole, gesättigte und ungesättigte C8-18-Fettalkohole, gesättigte und ungesättigte C8-18 Fettsäuren, Kohlenwasserstoffe und Kohlenwasserstoffmischungen, Fettsäureester aus C3-19 Fettsäuren und Cl-6-Alkylmonoolen, Dicarbonsäuredieester aus C4-8-Dicarbonsäuren und C1- 6-Alkylmonoolen, und Mischungen dieser Stoffe.

Penetrationsverstärkende Mittel verbessern den Flux des Wirkstoffes durch die Haut, auf welche das TTS aufgebracht ist. Beispiele aus den vorgenannten Stoffen sind: 1,2-Propandiol, Menthol, Dexpanthenol, Benzylalkohol, Laurylalkohol, Isocetylalkohol, Cetylalkohol, Mineralöl, Laurinsäure, Isopalmitinsäure, Isostearinsäure, Ölsäure; Methylester, Ethylester, 2-Hydroxyethylester, Glycerolester, Propylester, Isopropylester, Butylester, sec.-Butylester oder Isobutylester der Laurinsäure, Myristinsäure, Stearinsäure oder Palmitinsäure. Bevorzugt ist der Einsatz von Dimethylisosorbid, Isopropylmyristat und Laurylalkohol, höchstbevorzugt von Laurylalkohol. Als weitere Hilfsstoffe kommen beispielsweise Kristallisationsinhibitoren in Frage. Als Kristallisationsinhibitoren sind hochdisperses Siliciumdioxid oder makromolekulare Stoffe wie Polyvinylpyrrolidone, Polyvinylalkohole, Dextrine, Dextrane, Sterine, Gallensäuren und insbesondere Vinylpyrrolidon-Vinylacetat-Copolymere geeignet wie Kollidon®VA 64. Es versteht sich, dass jedenfalls das penetrationsverstärkende Mittel ebenfalls ausreichend durch die Matrix bzw. die Diffusionsbarriere diffundieren muss. Im Falle des Einsatzes einer Matrix sowie des Hilfsstoffes Laurylalkohol bildet der Laurylalkohol vorzugsweise

10 bis 30 Gew.-%, höchstvorzugsweise 15 bis 20 Gew.-%, der Matrix.

Neben den hier aufgeführten Bestandteilen werden der Matrix-Formulierung noch als Antioxidantien schwefelhaltige Aminosäuren wie Cystein, Methyldonatoren wie Methionin oder Antioxidantien wie Glutathion oder Natriumbisulfit in ausreichender Menge beigefügt, da sich in den Untersuchungen überraschend gezeigt hat, dass sich hierdurch die Bildung toxischer Oxidationsprodukte des Lisurids, wie z. B. des Lisurid-N-oxids, verhindern oder stark reduzieren lässt. Antioxidantien wie Glutathion können darüber hinaus bei Morbus Parkinson durchaus synergistisch wirken, da bei dieser Krankheit oxidativer Stress eine erhebliche Rolle spielt und schon in sehr frühen Stadien ein Glutathionmangel in der dopaminergen Substantia nigra bekannt ist. Methionin wiederum ist als Methyldonator besonders erwünscht, da der Levodopa-Abbau besonders durch 0-Methylierung stattfindet (COMT) und hierdurch bei den erforderlichen Levodopa-Mengen (bis im Grammbereich eine Tagesdosis) ein Homoserin-Anstieg entsteht, der als Risikofaktor für kardiale und zerebrale Ereignisse gilt.

Die Hilfsstoffe können grundsätzlich 0 bis 50 Gew.-% der Matrix bilden. Der Wirkstoff kann 0,2 bis 20 Gew.-%, vorzugsweise 1 bis 10 Gew.-%, der Matrix bilden. Die Summe der Anteile an Matrixhauptkomponente, Hilfsstoffen und Wirkstoffen bildet dabei stets 100 Gew.-%.

Die Dosis des Wirkstoffes in einem das TTS tragenden menschlichen Körper hängt neben den vorstehenden diffusionsbezogenen Eigenschaften des TTS auch von dessen wirksamer Fläche mit der Haut ab. Wirksame Fläche meint hierbei die Fläche, mit welcher die Matrix oder die Diffusionsbarriere an der Haut anzuliegen kommt. Vorzugsweise erfolgt die Variation nach Massgabe der gewünschten Dosis in einem Bereich von 1 bis 100 $cm^2$.

Im Rahmen der Erfindung lassen sich dabei, bei abgestimmten Flux für eine vorgegebene Indikation, leicht patientenindividuelle Dosisvariationen von einem Arzt einrichten, nämlich durch Wahl einer geeigneten Grösse. Somit kann die Behandlung beispielsweise auf unterschiedliche Körpergewichte, Altersgruppen etc. unschwer abgestellt werden.

Insbesondere ist es möglich, ein TTS, welches eine (eher grosse) Standardfläche aufweist, mit Unterteilungsmarkierungen für Teildosen auszustatten, so dass ein Anwender lediglich einen einer bestimmten Dosis entsprechenden Teilabschnitt abtrennen und anwenden kann. Entsprechende Aufdrucke lassen sich unschwer auf der Deckschicht anbringen.

Die Anwendung des Wirkstoffs Lisurid, seiner Salze bzw. Derivate mit ähnlich günstigen Eigenschaften hat die folgenden therapeutischen Vorzüge:

- Infolge ihrer ausserordentlich starken Affinität für Dopamin-und andere Monoamin-Rezeptoren lassen sich diese Substanzen extrem niedrig dosieren (im Falle von Lisurid: effektive Tagesdosierungen ab 0,075 mg p. o., bei hohem First-pass-Effekt) und damit lässt sich mit einem TTS bei sehr geringer Applikationsfläche sehr einfach ein wirksamer und über die Fläche gut kontrollierbarer Wirkstoffspiegel über 24 Stunden und länger erzielen

- Im Gegensatz aber zu langwirkenden oralen Wirkstoffen, wie z. B. Cabergolin, ist bei der transdermalen Dosierung von Lisurid und vergleichbaren Wirkstoffen nicht nur eine wesentlich bessere Dosierbarkeit gegeben (Wegfall des erheblichen und sehr variablen First-pass Effekts nach oraler Gabe z. B. von Cabergolin), sondern die Wirkungen können auch bei Bedarf (z. B. beim Auftreten von Nebenwirkungen) sehr rasch durch Entfernen des Pflasters beseitigt werden. Danach kommt die sehr kurze terminale Halbwertszeit von Lisurid im Blut (ca. 2 h) zum Tragenganz im Gegensatz zu den Tage anhaltenden Nebenwirkungen einmal eingenommener oraler Dopaminagonisten.

Durch die Kombination dieser Effekte ist es überraschend gelungen, die Vorteile kontinuierlicher und langer dopaminerger Stimulation mit den anderen Vorzügen kurz wirkender dopaminerger Pharmaka in einer Anwendung zu kombinieren.

Die Kombination dieser Eigenschaften erlaubt es auch, je nach individueller Situation und Bedarf des Patienten in einer "massgeschneiderten" Anwendung durch die Möglichkeiten der Wahl des Applikationsschemas zweier Pflaster (Entfernen und Neuanlegen gleichzeitig, überlappend oder mit Intervall) oder, noch besser, durch die Modifikation der Anflutungsphase der TTS-Formulierung fast beliebige zirkadiane Rhythmen der dopaminergen Therapie zu erreichen:

A Kontinuierliche Stimulierung, wenn Anflutungsphase des Pflasters der terminalen Halbwertszeit nach Pflaster-Entfernung entspricht ($tmax \approx t/2$, gegebenenfalls kurzes Intervall, oder bei gleichzeitiger Neuapplikation eines TTS mit relativ rascher Anflutung)

B Eine Phase mit verstärkter Stimulierung (z. B. bei Therapie-Einstellung oder zur Überbrückung einer "off"-Phase des Patienten) durch Applikation eines zweiten Pflasters, solange das erste noch auf der Haut ist, oder durch Pflaster mit sehr rascher Anflutung ($tmax << t/2$) oder sehr langsamem Abfluten (z. B. bei kleiner Fläche und zunehmender Wirkstoff-Diffusion mit Verringerung des Konzentrationsgradienten), sowie

C Eine Phase mit verminderter dopaminerger Stimulierung, z. B. zum Vermindern Tageszeit spezifischer Nebenwirkungen, entweder durch Einhalten eines Intervalls zwischen Pflaster Abnahme und Anlegen des neuen Pflasters, oder, einfacher, durch die gleichzeitige Verwendung eines neuen Pflasters mit sehr langsamer Anflutung (tmax >> t/2) zum Zeitpunkt der Abnahme.

Insgesamt bestehen hier also überraschenderweise bei der Anwendung nur eines Wirkstoffes mit geeigneter Rezeptor-Affinität, Wirksamkeit und Kinetik alle Möglichkeiten einer für den Patienten einfach anzuwendenden und gut steuerbaren dopaminergen Therapie. Da hierdurch Nebenwirkungen vermieden werden, wie sie bei den bisherigen oralen und transdermalen Therapien fast unvermeidbar sind, lässt sich auch eine stärkere Wirksamkeit bzw. ein deutlich besserer therapeutischer Effekt mit einfachen Mitteln erreichen.

Dies bedeutet, dass sich die Levodopa-Therapie mit ihren Langzeit-Komplikationen vermeiden oder hinauszögern lässt bzw. dass sie bzw. andere orale dopaminerge Therapien nur niedrig dosiert gegeben werden müssen und damit besser verträglich sind.

In diesen Zusammenhängen lehrt die Erfindung auch ein TTS-Set zur Behandlung der Erreichung und Erhaltung einer kontinuierlichen Rezeptor-Stimulation mit circadianem Rhythmus insbesondere bei der Parkinsonschen Krankheit, wobei das Set eine Mehrzahl von TTS-Elementen enthält, wobei die Elemente zur Abgabe von unterschiedlichen Dosen eingerichtet sind.

Hierzu können die TTS Elemente voneinander separiert sein, wobei die TTS Elemente mit einer kontinuierlich ansteigenden Folge für F im Bereich von 0,1 bis 5 $\mu g/cm^2/h$ eingerichtet sind. Zusätzlich oder unabhängig hiervon können die TTS Elemente in kontinuierlicher Folge mit unterschiedlicher wirksamer Fläche ausgestattet sind. In letzterem Fall ist es möglich, mit einheitlichen F-Werten zu arbeiten. Dann können TTS-Elemente durch die anzuwendenden Flächen indizierende Markierungen auf einem grossen TTS Konstrukt gebildet sein. Selbstverständlich ist auch eine bereits separierte Ausführungsform möglich.

Die Erfindung ist auch für andere Indikationen einsetzbar. Eine Einsatzmöglichkeit ist ein TTS-Set zur Behandlung oder Prävention des prämenstruellen Syndroms und seiner Symptome, wobei F vorzugsweise von 0,1 bis 0,5 $\mu g/cm^2/h$ beträgt, sowie ein TTS-Set zur Lactationshemmung, wobei F vorzugsweise von 0,1 bis 0,5 $\mu g/cm^2/h$ beträgt.

Beispiel 1 : Flux Messung

Zur Flux-Messung wird eine FRANZ Durchfluss Diffusionszelle verwendet. Die Messfläche beträgt 2 cm2. Als Hautprobe werden 4 cm2 ventrale und dorsale Haut einer männlichen haarlosen Maus (MF1 hr/hr Ola/Hsd, erhältlich von Harlan Olac, UK) verwendet, wobei subkutanes Fettgewebe sorgfältig entfernt wird.

Auf die eingesetzte Haut ist ein 2 cm2 TTS appliziert. Gegenüberliegend ist das Akzeptormedium angeordnet. Es ist verdünntes HHBSS (Hepes Hanks Balanced Salt Solution) enthaltend 5,96 g/l Hepes, 0,35 g/l $NaHCO_3$ und 0,1 ml/l 10x HBSS (erhältlich von Gibco, Eggenstein, DE). Weiterhin sind 1000 I.E./ml Penicillin (Benzylpenicillin Kaliumsalt, erhältlich von Fluka, Neu-Ulm, DE).

Die Messung erfolgt im einzelnen wie folgt. Das zu messende TTS wird zunächst auf die Haut appliziert. Sofort danach wird die Haut in die Diffusionszelle montiert. Das Akzeptor-Medium wird in Intervallen von 2h zwischen t=0 und t=6 h und von 8 h zwischen t=6 h und t=54h beprobt. Pro Stunde werden 1ml Akzeptor Medium durch die Diffusionszelle mittels einer peristaltischen Pumpe gepumpt. Die Temperatur des Akzeptor-Mediums wird mittels eines zirkulierenden Wasserbades kontrolliert und hält die Oberfläche der Haut auf einer Temperatur von 31°C mit 1 °C Genauigkeit.

Die Wirkstoffkonzentration in dem Akzeptor-Medium wird gemäss folgender Details mittels eines Radioimmunoassays bestimmt.

Kalibrierungskurven: Diese werden unter Verwendung von zwei unterschiedlichen Methanollösungen von nicht radioaktivem Lisuridhydrogenmaleatsalz, enthaltend je 1 mg/ml, konstruiert. Diese Lösungen werden unterschiedlich mit BSA-Puffer (0,041 M $Na_2HP0_2$*$2H_20$, 0,026 M $KH_2PO_4$, 0,154 M NaCl, 0,015 M $NaN_3$, 0,1% (w/v) BSA, pH 7, supplementiert mit 0,05% (w/v) Ascorbinsäure) verdünnt, um Lisurid free base Konzentrationen im Bereich von 1000-3,9 pg/0,1ml zu erhalten. Zusätzlich wird eine wirkstoffreie Probe (0pg) eingesetzt. Die Kalibrierungsproben werden dreifach analysiert. Die Lisurid Konzentrationen werden mittels der pharmacokinetic RIO PC Software, 2.5, berechnet (andere übliche Software ist ebenfalls einsetzbar).

Probenpräparation: Vor der Analyse wird das Akzeptormedium mit BSA-Puffer verdünnt zwecks Einstellung von Konzentrationen im auswertbaren Bereich der Kalibrierungskurve. 100 $\mu$l verdünnte Probe werden direkt der radioimmunologischen Analyse unterzogen.

Antiserum: Das Antiserum (Kaninchen) ist erhältlich durch Immunisierung mit dem Immunogen Lisurid-1- succinyl-BSA. Die Verdünnung des Antiserums im Assay ist 1 : 12500.

Tracer: $^3H$-Lisuridhydrogenmaleat mit einer spezifischen Aktivität von 4,3 GBq/mg wird verwendet.

Inkubation: zu 0,7 ml BSA-Puffer werden 0,1 ml BSA Puffer mit Wirkstoff, 0,1 ml Tracerlösung (ca. 5000 cpm/ 0, 1 ml BSA-Puffer) und 0,1 ml verdünntes Antiserum (1 : 12500) gegeben und es wird für 18 h bei 4°C inkubiert.

Separierung: antikörpergebundenes Lisurid wird von freiem durch Zugabe von 0,2 ml Holzkohlesuspension (1,25% (w/v) und 0,125% (w/v) Dextran in BSA-Puffer) und Inkubation für 30 min. bei 0°C getrennt. Die Holzkohle wird durch Zentrifugation bei 3000 g für 15 min. sedimentiert. Der Überstand (enthaltend antikörpergebundenen Wirkstoff) wird dekantiert und der radiometrischen Analyse zugeführt.

Radiometrische Analyse: Zum Überstand werden 4ml des Szintillations Cocktails Atomlight (NEN) gegeben. Die Zählung erfolgt mit einem WALLAC 1409 oder 1410 β-Szintillationszähler ohne quench control.

Auswertung: Der perkutane Haut Flux wird wie folgt berechnet:

$$F = (C * R)/ (A * T),$$

wobei F den percutanen Flux [ng/cm$^2$/h], C die Wirkstoffkonzentration im Akzeptormedium [ng/ml], R den Akzeptormediumsfluss [1ml/h], A die Messfläche [2cm$^2$] und T das Beprobungszeitintervall [h] sind.

Maximaler transdermaler Wirkstoffflux wird direkt von den Daten genommen. Mittlere perkutane Fluxwerte werden während Tag 1 und Tag 2 des Experiments bestimmt, basierend auf der kumulativ absorbierten Dosis in dem Zeitintervall t=0-22 und t=22-54.

Angaben für die Herstellung von TTS

Beispiel 2: TTS A

15 mg Kollidon VA 64 (Kristallisationsinhibitor) werden in 15 mg Isopropanol gelöst. Dann werden 5 mg Lisurid eingestreut. 80mg Polyacrylatkleber (Gelva 7881) werden in einem Becherglas vorgelegt und die vorstehende Suspension, unter Nachspülen mit 30 mg Isopropanol, hinzugegeben. Nach gründlicher Durchmischung wird das erhaltene kristallfreie Wetmix mit 500 μm Rakel auf einem silikonisierten Liner ausgezogen. Dann wird bei 60°C für 20 min. getrocknet und schliesslich eine Deckschicht auflaminiert.

Messungen des Flux gemäss Beispiel 1 ergeben für F einen Tag 1 Wert von 0,43, einen Tag 2 Wert von 0,44 und einen maximalen F von 0,85 (jeweils in μg/cm$^2$/h).

Beispiel 3: TTS B

12,5 mg Dimethylisosorbid werden mit 2 mg Lisurid in 15 mg Isopropanol suspendiert. 80 mg Polyacrylatkleber (Gelva 7881) werden in einem Becherglas vorgelegt und die vorstehende Suspension, unter Nachspülen mit 30 mg Isopropanol, hinzugegeben. Nach gründlicher Durchmischung wird das erhaltene kristallfreie Wetmix mit 500 μm Rakel auf einem silikonisierten Liner ausgezogen. Dann wird bei 60°C für 20 min. getrocknet und schliesslich eine Deckschicht auflaminiert.

Messungen des Flux gemäss Beispiel 1 ergeben für F einen Tag 1 Wert von 0,23, einen Tag 2 Wert von 0,28 und einen maximalen F von 0,50 (jeweils in μg/cm$^2$/h).

Beispiel 4: TTS C

27,2 mg Polyvinylpyrrolidon (Kristallisationsinhibitor) und 16,3 mg Laurylalkohol werden bei 60°C gelöst. Dann werden 2 mg Lisurid und 0,5 mg Glutathion in dieser Lösung bei 60°C gelöst. 39,38 mg Eudragit E100,13,41 mg Citroflex 4A und 1,71 mg Bernsteinsäure werden bei 150-200°C geschmolzen. Nach Abkühlung auf 80°C wird die Lisuridlösung unter Rühren hinzugegeben. Bei 80 C wird mit 500 μm Rakel auf einem silikonisierten Liner ausgezogen. Dann wird auf 20°C abgekühlt und schliesslich ggf. eine Deckschicht auflaminiert.

Messungen des Flux gemäss Beispiel 1 ergeben für F einen Tag 1 Wert von 0,90, einen Tag 2 Wert von 1,6 und einen maximalen F von 2,4 (jeweils in μg/cm$^2$/h).

## Patentansprüche

1. Set aus transdermalen therapeutischen Systemen (TTS-Set), wobei das Set eine Mehrzahl von TTS-Elementen enthält und wobei die Elemente zur Abgabe von unterschiedlichen Dosen eingerichtet sind, wobei das transdermale therapeutische System (TTS) eine Arzneimittelschicht aufweist, welche zumindest eine einen Wirkstoff enthaltende Matrix und/oder ein Wirkstoffreservoir und hautseitig des Wirkstoffreservoirs eine wirkstoffpermeable Diffusionsbarriere sowie als Wirkstoff ein Ergolin-Derivat gemäss Formel I oder dessen physiologisch verträgliches Salz mit einer Säure enthält,

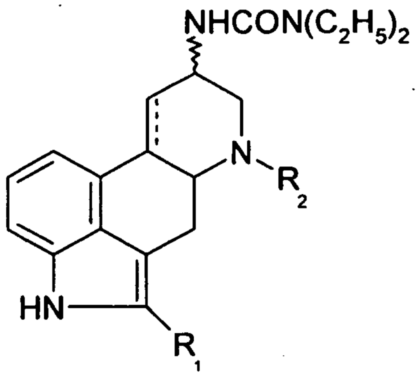

Formel I

worin ----- eine Einfachbindung oder eine Doppelbindung ist, worin R1 ein H-Atom oder ein Halogenatom, insbesondere ein Bromatom ist, und worin R2 C1-4-Alkyl ist, zur Erreichung und Erhaltung des circadianen Rhythmus unter kontinuierlicher dopaminerger Therapie,
wobei die Matrix und/oder die Diffusionsbarriere ausgewählt ist mit der Massgabe, dass der transdermale Flux F durch Humanhaut im Bereich von 0,1 bis 5,0 $\mu g/cm^2/h$ liegt.

**2.** TTS-Set nach Anspruch 1, wobei die TTS-Elemente voneinander separiert sind und wobei die TTS Elemente mit einer kontinuierlich ansteigenden Folge für F im Bereich von 0,1 bis $5 \mu g/cm^2/h$ eingerichtet sind.

**3.** TTS-Set nach Anspruch 1 oder 2, wobei die TTS Elemente in kontinuierlicher Folge mit unterschiedlich wirksamer Fläche ausgestattet sind.

**4.** TTS-Set nach einem der Ansprüche 1 bis 3, wobei das Ergolin-Derivat Lisurid oder dessen physiologisch verträgliches Salz ist.

**5.** TTS-Set nach einem der Ansprüche 1 bis 4, wobei auf der hautabgewandten Seite der Matrix und/oder des Wirkstoffreservoirs eine Deckschicht angeordnet ist.

**6.** TTS-Set nach einem der Ansprüche 1 bis 5, wobei die Matrix und/oder Diffusionsbarriere einen Stoff, ausgewählt aus der Gruppe bestehend aus Polyacrylat, Polyurethan, Celluloseether, Silikon, Polyvinylverbindungen, Silikat und Mischungen dieser Stoffe sowie Copolymere dieser Polymerverbindungen, vorzugsweise hydrophiles Polyacrylat mit basischen Substituenten, als Hauptmatrixkomponente aufweist.

**7.** TTS-Set nach einem der Ansprüche 1 bis 6, wobei die Diffusionsbarriere ein synthetisches Polymer ausgewählt aus der Gruppe bestehend aus Celluloseester, Celluloseether, Silikon, Polyolefin und Mischungen sowie Copolymere dieser Stoffe als Hauptbarrierenkomponente aufweist.

**8.** TTS-Set nach einem der Ansprüche 1 bis 7, wobei die Matrix und/oder das Wirkstoffreservoir und/oder die Diffusionsbarriere ein penetrationsverstärkendes Mittel enthält, welches vorzugsweise ausgewählt ist aus der Gruppe bestehend aus C1-C8 aliphatischen, cycloaliphatischen und aromatischen Alkoholen, gesättigten und ungesättigten C8-18-Fettalkoholen, gesättigten und ungesättigten C8-18-Fettsäuren, Kohlenwasserstoffen und Kohlenwasserstoffmischungen, Fettsäureestern aus C3-19-Fettsäuren und C1-6- Alkylmonoolen, Dicarbonsäurediestern aus C4-8-Dicarbonsäuren und C1-6-Alkylmonoolen, und Mischungen dieser Stoffe.

**9.** TTS-Set nach einem der Ansprüche 1 bis 8, wobei die Matrix und/oder das Wirkstoffreservoir und/oder die Diffusionsbarriere einen Kristallisationsinhibitor enthält, welcher ausgewählt ist aus der Gruppe bestehend aus hochdispersem Siliciumdioxid oder makromolekularen Stoffen wie Polyvinylpyrrolidone, Polyvinylalkohole, Dextrine, Dextrane, Sterine, Gallensäuren und insbesondere Vinylpyrrolidon-Vinylacetat Copolymere.

**10.** TTS-Set nach einem der Ansprüche 1 bis 9, wobei die Matrix und/oder das Wirkstoffreservoir und/oder die Diffusionsbarriere ein Antioxidant enthält, welches ausgewählt ist aus der Gruppe bestehend aus schwefelhaltige Aminosäuren wie Cystein, Methyldonatoren wie Methionin oder Antioxidantien wie Glutathion oder Natriumbisulfit.

**11.** TTS-Set nach einem der Ansprüche 1 bis 10 zur Behandlung oder Prävention des prämenstruellen Syndroms, wobei F vorzugsweise von 0,1 bis 0,5µg/cm²/h beträgt.

**12.** TTS-Set nach einem der Ansprüche 1 bis 11 zur Lactationshemmung, wobei F vorzugsweise von 0,1 bis 0,5µg/cm²/h beträgt.

**Claims**

**1.** Set of transdermal therapeutic systems (TTS set), wherein the set contains a multitude of TTS elements and wherein the elements are configured for releasing different doses, wherein the transdermal therapeutic system (TTS) has a pharmaceutical layer containing at least one matrix having an active ingredient, and/or an active ingredient reservoir and a diffusion barrier which is permeable to active ingredients and which is arranged on the skin side of the active ingredient reservoir; and an ergoline derivative according to formula I or a physiologically compatible salt thereof with an acid as the active ingredient,

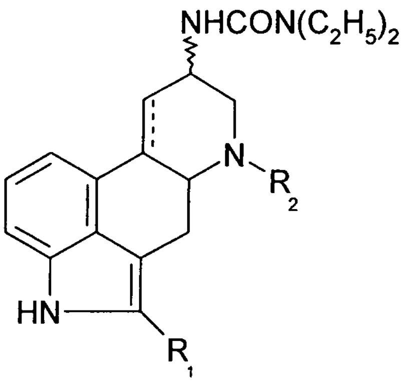

Formula I

wherein ---- is a single or double bond, wherein R1 is an H atom or a halogen atom, particularly a bromine atom, and wherein R2 is C1-4 alkyl, for obtaining and maintaining the circadian rhythm under a continuous dopaminergic therapy,
wherein the matrix and/or the diffusion barrier are selected so that the transdermal flux F through human skin is in the range from 0.1 to 5.0 µg/cm²/h.

**2.** The TTS set according to claim 1 wherein the TTS elements are separated from each other and wherein the TTS elements are configured for a continuously ascending sequence of F ranging from 0.1 to 5 µg/cm²/h.

**3.** The TTS set according to claims 1 or 2 wherein the TTS elements are equipped with different active surfaces in a continuous sequence.

**4.** The TTS set according to any one of claims 1 to 3 wherein the ergoline derivative is lisuride or a physiologically compatible salt thereof.

**5.** The TTS set according to any one of claims 1 to 4 wherein a covering layer is provided on the side of the matrix and/or the active ingredient reservoir that faces away from the skin.

**6.** The TTS set according to any one of claims 1 to 5 wherein the matrix and/or diffusion barrier comprise as their main matrix component a substance selected from the group consisting of polyacrylate, polyurethane, cellulose ether, silicone, polyvinyl compounds, silicate and mixtures of these substances as well as copolymers of these polymeric compounds, preferably hydrophilic polyacrylate with basic substituents.

7. The TTS set according to any one of claims 1 to 6 wherein the diffusion barrier comprises as its main barrier component a synthetic polymer selected from the group consisting of cellulose ester, cellulose ether, silicone, polyolefin and mixtures as well as copolymers of these substances.

8. The TTS set according to any one of claims 1 to 7 wherein the matrix and/or the active ingredient reservoir and/or the diffusion barrier contains a penetration-enhancing agent that is preferably selected from the group consisting of C1-C8 aliphatic, cycloaliphatic and aromatic alcohols, saturated and unsaturated C8-18 fatty alcohols, saturated and unsaturated C8-18 fatty acids, hydrocarbons and hydrocarbon mixtures, fatty acid esters from C3-19 fatty acids and C1-6-alkyl monools, dicarboxylic acid diesters from C4-8-dicarboxylic acids and C1-6 alkyl monools, and mixtures of these substances.

9. The TTS set according to any one of claims 1 to 8 wherein the matrix and/or the active ingredient reservoir and/or the diffusion barrier contains a crystallization inhibitor that is selected from the group consisting of highly dispersed silicon dioxide or macromolecular substances such as polyvinyl pyrrolidone, polyvinyl alcohols, dextrines, dextranes, sterines, bile acids and, in particular, vinyl pyrrolidone vinylacetate copolymers.

10. The TTS set according to any one of claims 1 to 9 wherein the matrix and/or the active ingredient reservoir and/or the diffusion barrier contain an antioxidant that is selected from the group consisting of sulfur-containing amino acids such as cysteine, methyl donors such as methionine, or antioxidants such as glutathione or sodium hydrogensulfite.

11. The TTS set according to any one of claims 1 to 10 for the treatment or prevention of the premenstrual syndrome wherein the preferred F value is in the range from 0.1 to 0.5 $\mu g/cm^2/h$.

12. The TTS set according to any one of claims 1 to 11 for lactation inhibition wherein the preferred F value is in the range from 0.1 to 0.5 $\mu g/cm^2/h$.

**Revendications**

1. Kit de systèmes thérapeutiques transdermiques (kit TTS), le kit contenant une pluralité d'éléments TTS et les éléments étant installés pour délivrer différentes doses, le système thérapeutique transdermique (TTS) comportant une couche de produits pharmaceutiques qui comprend au moins une matrice contenant un principe actif, et/ou un réservoir de principe actif et du côté peau du réservoir de principe actif, une barrière de diffusion perméable au principe actif, ainsi qu'en tant que principe actif un dérivé d'ergoline selon la formule I ou son sel physiologiquement compatible,

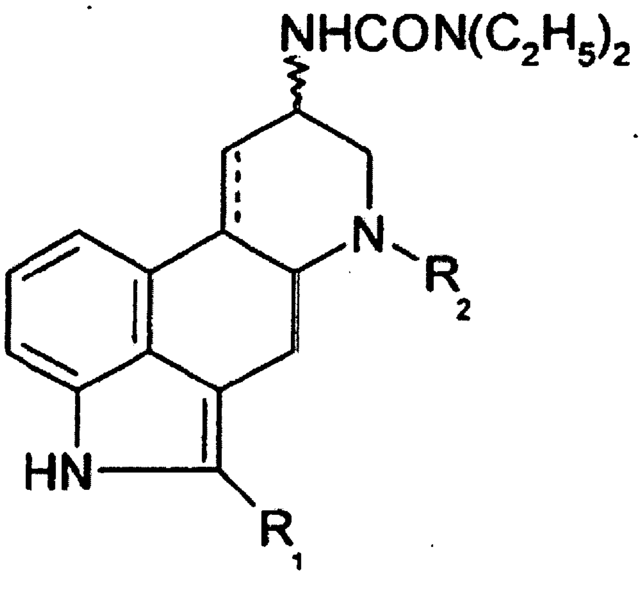

Formule I

Dans laquelle ------ est une liaison simple ou une liaison double, dans laquelle R1 est un atome H ou un atome halogène, notamment un atome de brome et dans laquelle R2 est un alkyle en C1-4 pour accéder au et obtenir le rythme circadien sous thérapie continue à la dopamine,
la matrice et/ou la barrière de diffusion étant choisie selon le critère que le flux transdermique X à travers la peau humaine soit de l'ordre de 0,1 à 5,0 $\mu g/cm^2$.

2. Kit TTS selon la revendication 1, les éléments TTS étant séparés les uns des autres et les éléments TTS étant installés avec une séquence continuellement croissante pour F, de l'ordre de 0,1 à 5$\mu g/cm^2/h$.

3. Kit TTS selon la revendication 1 ou 2, les éléments TTS étant équipés dans une séquence continue d'une surface différemment active.

4. Kit TTS selon l'une quelconque des revendications 1 à 3, le dérivé d'ergoline étant du lisuride ou son sel physiologiquement compatible.

5. Kit TTS selon l'une quelconque des revendications 1 à 4, une couche de recouvrement étant disposée sur la face opposée à la peau de la matrice et/ou du réservoir de principe actif.

6. Kit TTS selon l'une quelconque des revendications 1 à 5, la matrice et/ou la barrière de diffusion comportant une substance, choisie dans le groupe comprenant le polyacrylate, le polyuréthane, l'éther cellulosique, le silicone, des composés polyvinyliques, du silicate et des mélanges desdites substances, ainsi que des copolymères desdits composés polymères, de préférence du polyacrylate hydrophile avec des groupes substituants basiques en tant que composante principale de la matrice.

7. Kit TTS selon l'une quelconque des revendications 1 à 6, la barrière de diffusion comportant un polymère synthétique, choisi dans le groupe comprenant l'ester cellulosique, l'éther cellulosique, le silicone, les polyoléfines et des mélanges, ainsi que des copolymères desdites substances en tant que composante principale de la barrière.

8. Kit TTS selon l'une quelconque des revendications 1 à 7, la matrice et/ou le réservoir de principe actif et/ou la barrière de diffusion contenant un produit renforçant la pénétration qui est chosi de préférence dans le groupe comprenant des alcools aliphatiques, cyclo-aliphatiques et aromatiques en C1 à C8, des alcools gras saturés et insaturés en C8 à 18, des acides gras saturés et insaturés en C8 à 18, des hydrocarbures et des mèlanges d'hydrocarbures, des esters d'acides gras à partir d'acides gras en C3 à 19 et de monoalcools d'alkyle en C1 à 6, des diesters d'acide dicarbonique à partir d'acides dicarboniques en C4 à 8 et de monoalcools en C1 à 6 et des mélanges desdites substances.

9. Kit TTS selon l'une quelconque des revendications 1 à 8, la matrice et/ou le réservoir de principes actifs et/ou la barrière de diffusion contenant un inhibiteur de cristallisation, qui est choisi dans le groupe comprenant du dioxyde de silicium fortement dispersé ou des substances comme les pyrrolidones de polyvinyle, les alcools de polyvinyle, la dextrine, le dextrane, la stérine, les acides biliaires, des copolymères vinylacétate/pyrrolidones de vinyle.

10. Kit TTS selon l'une quelconque des revendications 1 à 9, la matrice et/ou le réservoir de principes actifs et/ou la barrière de diffusion contenant un antioxydant qui est choisi dans le groupe comprenant les acides aminés sulfureux, comme la cystéine, les donneurs de méthyle comme la méthionine ou des antioxydants comme le glutathionne ou le bisulfite de sodium.

11. Kit TTS selon l'une quelconque des revendications 1 à 10 pour le traitement ou la prevention du syndrome prémenstruel, F correspondant de préférence à de 0,1 bis 0,5 $\mu g/cm^2/h$.

12. Kit TTS selon l'une quelconque des revendications 1 à 11 pour inhiber la lactation, F correspondant de préférence à de 0,1 bis 0,5 $\mu g/cm^2/h$.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9220339 A
- WO 9100746 A
- US 3953454 A
- EP 056358 A
- US 4379790 A